# EUROPEAN PATENT APPLICATION

(11) **EP 2 653 108 A1**
(43) Date of publication of application: **23.10.2013**
(21) Application number: 13156681.2
(22) Date of filing: 26.02.2013
(51) Int. Cl.: A61B 7/00, A61B 5/113, A61B 5/00

(54) **Apnea determining program, apnea determining apparatus, and apnea determining method**

(30) Priority: 19.04.2012 JP 2012096053
(71) Applicant: FUJITSU LIMITED, Kawasaki-shi, Kanagawa 211-8588 (JP)
(72) Inventor: Nishida, Asako, Kawasaki-shi, Kanagawa 211-8588 (JP)
(74) Representative: Lewin, David Nicholas

(57) **Abstract**

A portable terminal (1) includes a breath determining unit(31) that determines, when sounds having similar frequency components have been periodically detected in picked-up sounds, the detected sounds to be breath sounds. The portable terminal further includes a snore determining unit(32) that determines, sound pressure of a determined breath sound has exceeded a first level threshold, the breath sound to be a snore sound. The portable terminal further includes an apnea determining unit(33) that determines, when a silent interval has been detected between consecutive snore sounds determined, the detected silent interval to be an apnea state.

## Description

### FIELD

The embodiment discussed herein is related to an apnea determining program, an apnea determining apparatus, and an apnea determining method.

### BACKGROUND

In recent years, the number of patients with sleep apnea syndrome as well as the number of people at risk for sleep apnea syndrome tend to increase year by year. In the first place, the sleep apnea syndrome is a disease of which the symptoms can be relieved by early treatment. Therefore, it is important for patients with sleep apnea syndrome as well as people at risk for sleep apnea syndrome to be aware of the occurrence of an apnea sate during sleep.

So, in recent years, there has been developed, for example, a portable terminal with a function of detecting an apnea state during sleep. This portable terminal records sounds made by a subject during sleep and detects a snore sound in recorded sound data. Then, when having detected a silent interval lasting for a predetermined time between consecutive snore sounds, the portable terminal determines this silent interval to be an apnea state. Incidentally, the predetermined time is, for example, 10 to 120 seconds. Accordingly, using the portable terminal, a user of the portable terminal can recognize whether a state likely to be an apnea state has occurred during subject's sleep.
[Patent document 1] Japanese Laid-open Patent Publication No. 2009-219713
[Patent document 2] Japanese National Publication of International Patent Application No. 11-505146
[Patent document 3] Japanese Laid-open Patent Publication No. 2006-167427

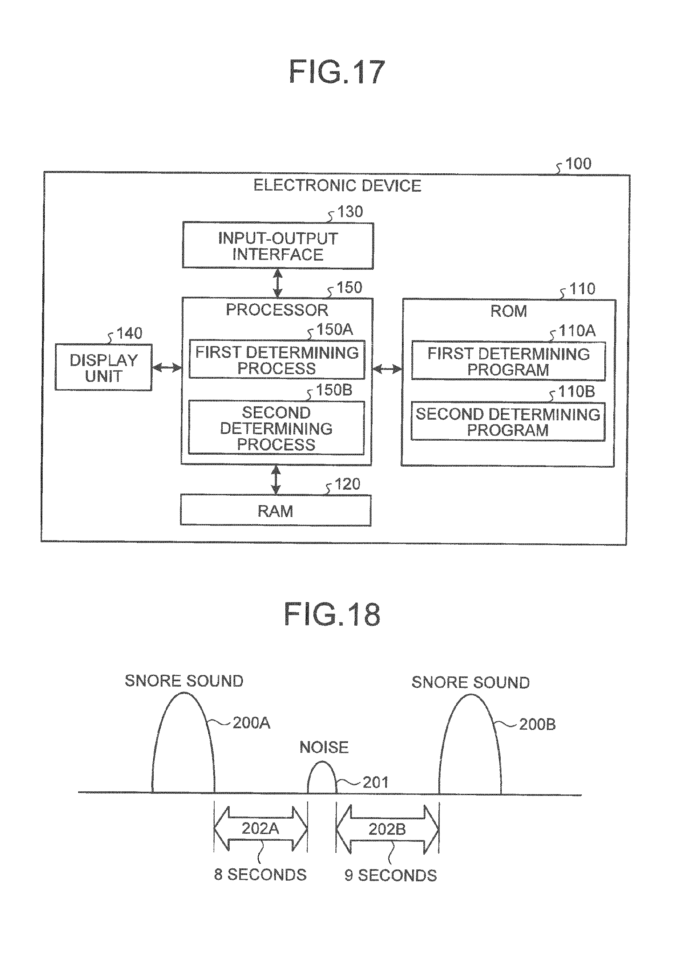

Incidentally, when a silent interval lasting for the predetermined time has been detected between consecutive snore sounds, the portable terminal determines the silent interval to be an apnea state. When a silent interval lasting for the predetermined time has not been detected, the portable terminal does not determine that an apnea state has occurred. FIG. 18 is an explanatory diagram illustrating an example of an apnea determining operation on the occurrence of a noise between snore sounds. In the example illustrated in FIG. 18, when a noise 201 occurred between consecutive snore sounds 200A and 200B, the noise 201 is incorrectly determined to be a snore. Therefore, in the example illustrated in FIG. 18, an actual silent interval is divided into an interval 202A between the snore sound 200A and the noise 201 and an interval 202B between the noise 201 and the snore sound 200B, and whether each of the silent intervals 202A and 202B lasted for a predetermined time is determined.

Consequently, despite the fact that the interval between the snore sounds 200A and 200B is a silent interval which is an apnea state, it is not determined to be a silent interval because, due to the occurrence of the noise 201, the divided intervals 202A and 202B do not meet a condition of the predetermined time (for example, 10 seconds); therefore, the portable terminal may fail to determine the apnea state.

One aspect of the present invention is to provide an apnea determining program, an apnea determining apparatus, and an apnea determining method capable of determining a state likely to be an apnea state with a high degree of accuracy.

Accordingly, it is an object in one aspect of an embodiment of the invention to provide an apnea determining program, an apnea determining device, and an apnea determining method capable of determining a state likely to be an apnea state with a high degree of accuracy.

### SUMMARY

According to an aspect of the embodiments, an apnea determining program causes a processor of an electronic device to execute: first determining, when having detected sounds having similar frequency components periodically in picked-up sounds, the detected sounds to be breath sounds; and second determining, when having detected a silent interval between consecutive breath sounds determined, the detected silent interval to be an apnea state.

### BRIEF DESCRIPTION OF DRAWINGS

FIG. 1 is an explanatory diagram illustrating an example of a portable terminal according to a present embodiment;
FIG. 2 is an explanatory diagram illustrating an example of a functional configuration of a processor;
FIG. 3 is an explanatory diagram illustrating an example of the operation of a breath determining unit;
FIG. 4 is an explanatory diagram illustrating an example of the operation of a snore determining unit;
FIG. 5 is an explanatory diagram illustrating an example of the operation of an apnea determining unit;
FIG. 6 is an explanatory diagram illustrating an example of the operation of a body motion determining unit (an example of an acoustic component);
FIG. 7 is an explanatory diagram illustrating another example of the operation of the body motion determining unit (an example of an acceleration component);
FIG. 8 is an explanatory diagram illustrating an example of a sleep log screen of the portable terminal;
FIG. 9 is a flowchart illustrating an example of the processing operation of the processor of the portable terminal involved in a breath determining process;
FIG. 10 is a flowchart illustrating an example of the processing operation of the processor of the portable terminal involved in a snore determining process;
FIG. 11 is a flowchart illustrating an example of the processing operation of the processor of the portable terminal involved in a body motion determining process;
FIG. 12 is a flowchart illustrating an example of the processing operation of the processor of the portable terminal involved in a first apnea determining process;
FIG. 13 is a flowchart illustrating an example of the processing operation of the processor of the portable terminal involved in a second apnea determining process;
FIG. 14 is a flowchart illustrating an example of the processing operation of the processor of the portable terminal involved in a sleep-advice providing process;
FIG. 15 is a flowchart illustrating an example of the processing operation of the processor of the portable terminal involved in a sleep-environment determining process;
FIG. 16 is a flowchart illustrating an example of the processing operation of the processor of the portable terminal involved in a hospital-site linking process;
FIG. 17 is an explanatory diagram illustrating an example of an electronic device that executes an apnea determining program; and
FIG. 18 is an explanatory diagram illustrating an example of an apnea determining operation on the occurrence of a noise between snore sounds.

### DESCRIPTION OF EMBODIMENTS

Preferred embodiments of the present invention will be explained with reference to accompanying drawings. However, the disclosed technique is not limited by these embodiments.

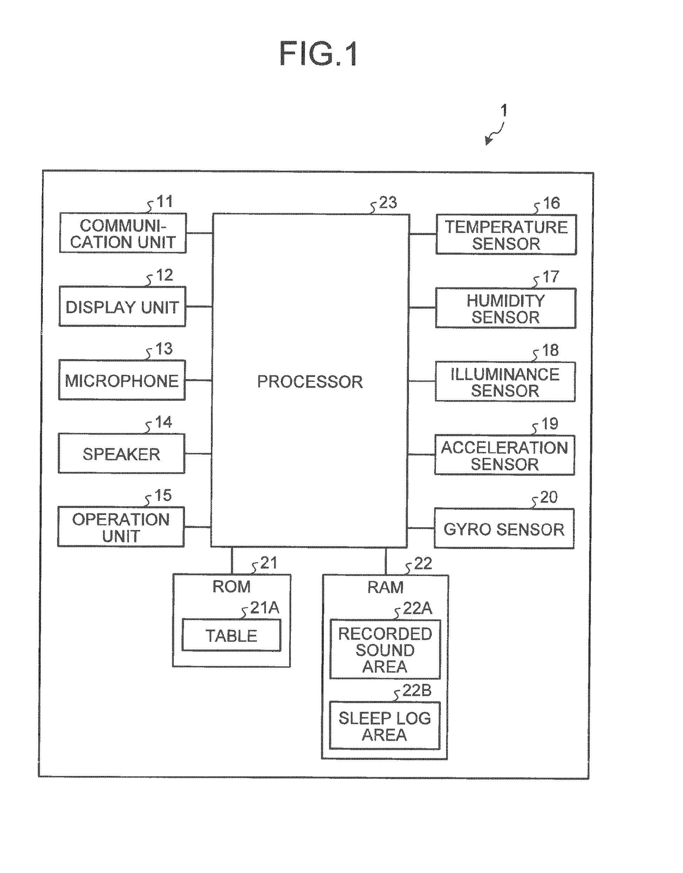

FIG. 1 is an explanatory diagram illustrating an example of a portable terminal according to the present embodiment. A portable terminal 1 illustrated in FIG. 1 includes a communication unit 11, a display unit 12, a microphone 13, a speaker 14, an operation unit 15, a temperature sensor 16, a humidity sensor 17, an illuminance sensor 18, an acceleration sensor 19, and a gyro sensor 20. The portable terminal 1 further includes a read-only memory (ROM) 21, a random access memory (RAM) 22, and a processor 23.

The portable terminal 1 is, for example, a cellular phone such as a smartphone. The communication unit 11 is an interface responsible for a radio function. The display unit 12 is, for example, a liquid crystal display (LCD) that displays thereon various information. The microphone 13 picks up a sound such as a voice. The speaker 14 externally outputs a sound. The operation unit 15 is, for example, a touch panel device that detects an operation made on an operation screen displayed on the display unit 12. Incidentally, as the touch panel device, for example, a resistive type is adopted; however, other types of touch panel devices, such as a surface acoustic wave type, an infrared type, an electromagnetic induction type, and a capacitance type, can also be adopted. The temperature sensor 16 measures, for example, temperature around the portable terminal 1. The humidity sensor 17 measures, for example, humidity around the portable terminal 1. The illuminance sensor 18 measures, for example, illuminance around the portable terminal 1.

The acceleration sensor 19 is a sensor that detects, for example, triaxial acceleration along the x-axis, the y-axis, and the z-axis of the portable terminal 1. The gyro sensor 20 is a sensor that detects, for example, triaxial angular velocity. The ROM 21 is a storage unit in which various programs, such as an apnea determining program, have been stored. Furthermore, a table 21A containing pieces of advice corresponding to various conditions to be described later has been stored in the ROM 21. In the RAM 22, various information is stored. The RAM 22 includes a recorded sound area 22A and a sleep log area 22B. The recorded sound area 22A is an area in which a sound during sleep picked up by the microphone 13 is stored as recorded sound data. The sleep log area 22B is an area in which various sleep logs to be described later are stored. The processor 23 controls the entire portable terminal 1.

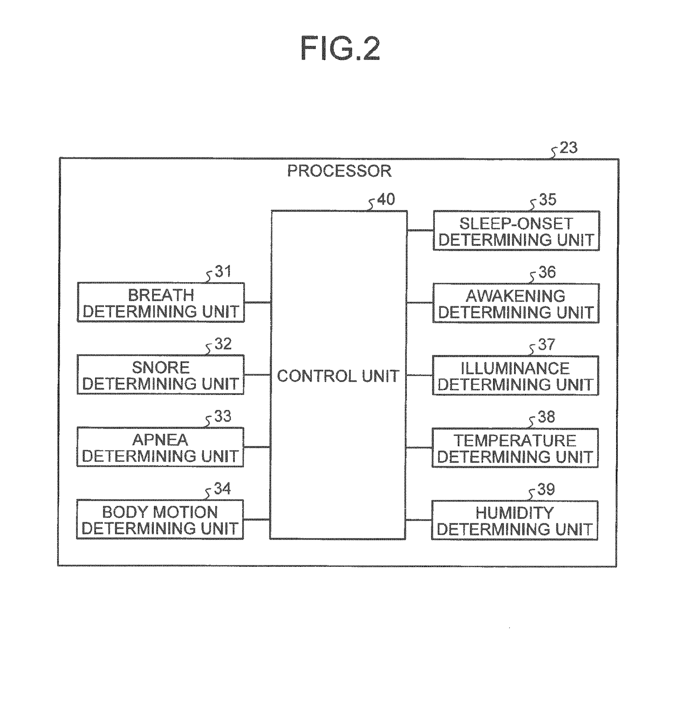

FIG. 2 is an explanatory diagram illustrating an example of a functional configuration of the processor 23. Incidentally, the processor 23 reads out the apnea determining program stored in the ROM 21, and makes up various processes as functions on the basis of the read apnea determining program. The processor 23 illustrated in FIG. 2 includes a breath determining unit 31, a snore determining unit 32, an apnea determining unit 33, a body motion determining unit 34, a sleep-onset determining unit 35, an awakening determining unit 36, an illuminance determining unit 37, a temperature determining unit 38, a humidity determining unit 39, and a control unit 40 as functions.

The control unit 40 controls the entire processor 23. Furthermore, once the activation of a sleep log function to be described below is started, the control unit 40 picks up a sound through the microphone 13, and stores the picked-up sound as recorded sound data in the recorded sound area 22A of the RAM 22. Incidentally, the sleep log function is a function of acquiring various logs relating to breath sounds, snores, apneas, and body motions with respect to each sleep.

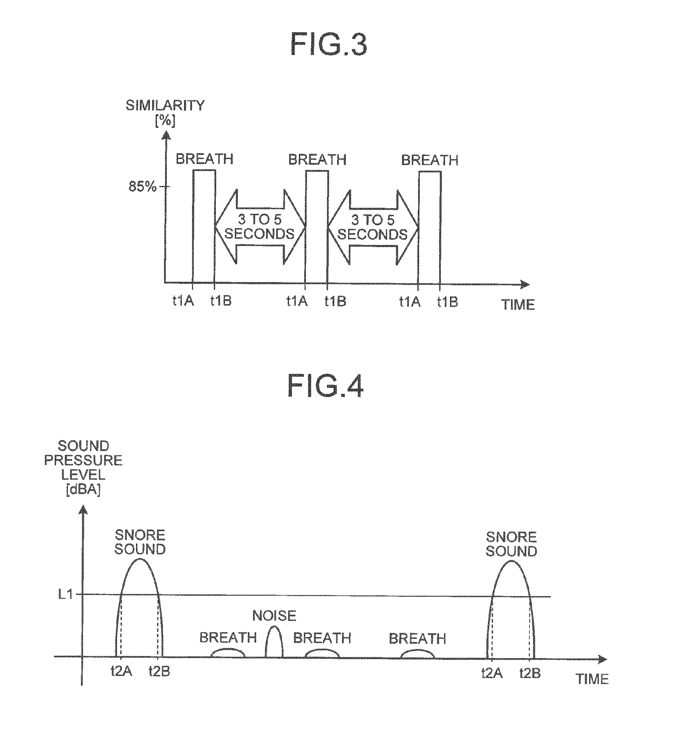

The breath determining unit 31 makes a power spectrum analysis of recorded sound data for one sleep stored in the recorded sound area 22A. Then, when having detected sounds having similar frequency components periodically on the basis of a result of the analysis of the recorded sound data, the breath determining unit 31 determines the sounds to be breath sounds of a subject, i.e., sounds of subject's breathing during sleep. Incidentally, the period differs among subjects; however, for convenience of explanation, it will be assumed that the period is every 3 to 5 seconds. FIG. 3 is an explanatory diagram illustrating an example of the operation of the breath determining unit 31. Focusing on a point that periodic subject's breathing during sleep, i.e., breath sounds have similar frequency components, the breath determining unit 31 sequentially determines a breath sound in recorded sound data on the basis of a degree of similarity of frequency components of a candidate breath sound.

The breath determining unit 31 sequentially specifies determining-target frequency components on the time axis of the recorded sound data. When a degree of similarity between the determining-target frequency components and the frequency components of the candidate breath sound has exceeded 85%, the breath determining unit 31 determines a sound of the determining-target frequency components to be start timing t1A of a breath sound. Furthermore, after having determined the start timing t1A of the breath sound, when a degree of the similarity between frequency components of the breath sound and the frequency components of the candidate breath sound has fallen to 85% or less, the breath determining unit 31 determines that it is stop timing t1B of the breath sound. Then, the breath determining unit 31 stores the start time of the breath sound at the start timing t1A of the breath sound and the stop time of the breath sound at the stop timing t1B of the breath sound in the sleep log area 22B of the RAM 22.

The snore determining unit 32 determines whether a sound pressure level of a breath sound determined by the breath determining unit 31 out of the recorded sound data for one sleep has exceeded a first level threshold L1. FIG. 4 is an explanatory diagram illustrating an example of the operation of the snore determining unit 32. When a sound pressure level of a breath sound has exceeded the first level threshold L1, the snore determining unit 32 determines that it is start timing t2A of a snore sound. Furthermore, after having determined the start timing t2A of the snore sound, when a sound pressure level of the breath sound has fallen to the first level threshold L1 or lower, the snore determining unit 32 determines that it is stop timing t2B of the snore sound. Incidentally, the first level threshold L1 is, for example, 40 dBA. Then, the snore determining unit 32 stores the start time of the snore sound at the start timing t2A of the snore sound, the stop time at the stop timing t2B of the snore sound, recorded sound data of the snore sound, and the number of hourly snores during the one sleep in the sleep log area 22B.

Furthermore, when the snore determining unit 32 has detected a sound at timing other than the timings of the breath sound determined by the breath determining unit 31, even if a sound pressure level of the sound has exceeded the first level threshold L1, the snore determining unit 32 determines the sound to be just a noise. Consequently, the snore determining unit 32 can avoid erroneous determination of a snore sound due to the occurrence of a noise between consecutive breath sounds.

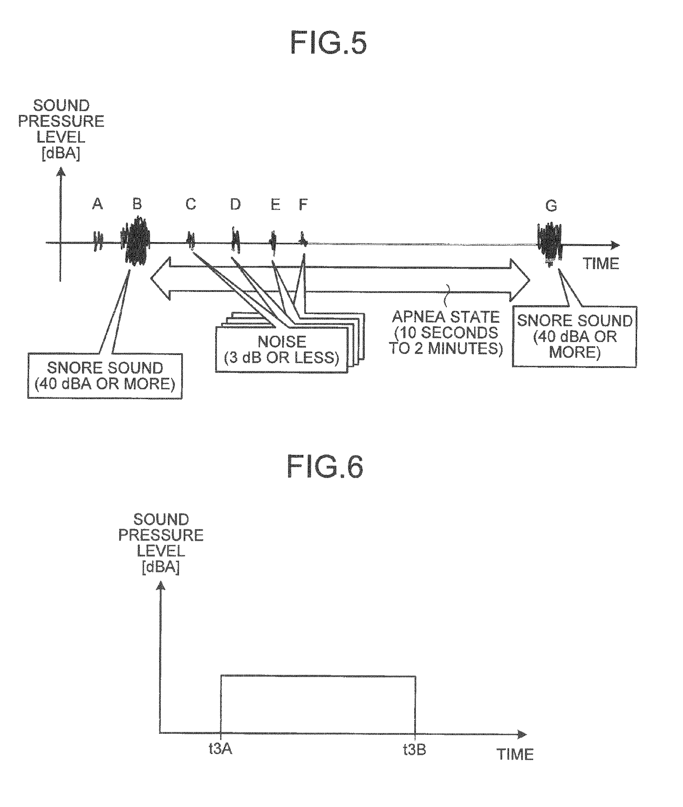

The apnea determining unit 33 determines, when a silent interval lasting for a predetermined time or more has been detected between snore sounds determined by the snore determining unit 32, the silent interval to be an apnea state. Incidentally, the silent interval is, for example, an interval in which a sound of less than 3 dB lasts for 10 seconds or more. FIG. 5 is an explanatory diagram illustrating an example of the operation of the apnea determining unit 33. The apnea determining unit 33 determines, when a silent interval has been detected between snore sounds, the silent interval to be an apnea state. Namely, the apnea determining unit 33 can determine an obstructive apnea of a snore sound → an apnea → a snore sound as illustrated in FIG. 5. The apnea determining unit 33 stores the start and stop times of the apnea state and the number of hourly apneas during the one sleep in the sleep log area 22B.

Furthermore, when a silent interval has been detected between breath sounds determined by the breath determining unit 31, the apnea determining unit 33 determines the silent interval to be an apnea state. Namely, the apnea determining unit 33 can determine the silent interval to be a central apnea of a breath sound → an apnea → a breath sound. Then, the apnea determining unit 33 stores the start and stop times of the apnea state and the number of hourly apneas during the one sleep in the sleep log area 22B.

The body motion determining unit 34 determines a body motion, such as subject tossing and turning, from an acoustic component during sleep and an acceleration component during sleep. FIG. 6 is an explanatory diagram illustrating an example of the operation of the body motion determining unit 34 (an example of an acoustic component). An acoustic component on the occurrence of a body motion is frequency components of a sound produced by a body motion, such as rustling of bedding caused by subject tossing and turning. When components of a sound produced by a body motion has been detected for a duration of a predetermined time or more in the recorded sound data for one sleep, the body motion determining unit 34 determines that the sound is due to a body motion. Then, the body motion determining unit 34 stores start time t3A of the body motion, stop time t3B of the body motion, and the number of hourly body motions during the one sleep in the sleep log area 22B.

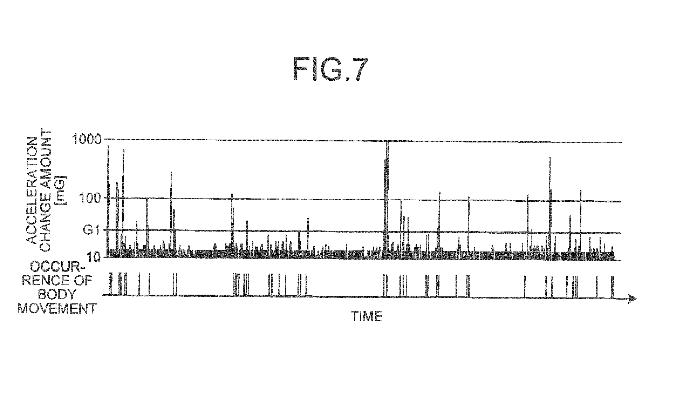

FIG. 7 is an explanatory diagram illustrating an example of the operation of the body motion determining unit 34 (an example of an acceleration component). An acceleration component on the occurrence of a body motion is what a vibration, which was caused by subject's tossing and turning, transmitted to the portable terminal 1 placed, for example, on the floor is represented as an acceleration change amount (unit: mG). When an acceleration change amount during sleep detected by the acceleration sensor 19 has exceeded a body motion threshold G1, the body motion determining unit 34 determines that to be due to a body motion. Then, the body motion determining unit 34 stores the start and stop times of the body motion and the number of hourly body motions during the one sleep in the sleep log area 22B.

The sleep-onset determining unit 35 determines whether a subject has fallen into a sleep state by using an AW2 sleep determining algorithm after the breath determining unit 31 has confirmed consecutiveness of breath sounds (sounds of subject's breathing during sleep). Then, when the subject has fallen into the sleep state, the sleep-onset determining unit 35 stores the sleep-onset time in the sleep log area 22B. The awakening determining unit 36 determines awakening on the basis of subject's action during sleep. Then, the awakening determining unit 36 stores the time at which the subject has fallen into an awakening state in the sleep log area 22B.

The illuminance determining unit 37 determines whether current illuminance detected by the illuminance sensor 18 is equal to or lower than a comfortable illuminance threshold. Incidentally, the comfortable illuminance threshold is, for example, 200 lx. When the current illuminance is equal to or lower than the comfortable illuminance threshold, the control unit 40 reads out illuminance advice for comfortable sleep according to this condition from the table 21A, and displays the read illuminance advice on the display unit 12. When the current illuminance is not equal to or lower than the comfortable illuminance threshold, the control unit 40 reads out illuminance advice to change the illuminance according to this condition, and displays the read illuminance advice on the display unit 12.

The temperature determining unit 38 determines whether current temperature detected by the temperature sensor 16 is within a comfortable temperature range. Incidentally, the comfortable temperature range is, for example, 16 to 20 degrees C; the setting of the comfortable temperature range can be adjusted according to the season. When the current temperature is within the comfortable temperature range, the control unit 40 reads out temperature advice for comfortable sleep according to this condition from the table 21A, and displays the read temperature advice on the display unit 12. When the current temperature is not within the comfortable temperature range, the control unit 40 reads out temperature advice to change the temperature according to this condition from the table 21A, and displays the read temperature advice on the display unit 12.

The humidity determining unit 39 determines whether current humidity detected by the humidity sensor 17 is within a comfortable humidity range. Incidentally, the comfortable humidity range is, for example, 45% to 55%; the setting of the comfortable humidity range can be adjusted according to the season. When the current humidity is within the comfortable humidity range, the control unit 40 reads out humidity advice for comfortable sleep according to this condition from the table 21A, and displays the read humidity advice on the display unit 12. When the current humidity is not within the comfortable humidity range, the control unit 40 reads out humidity advice to change the humidity according to this condition from the table 21A, and displays the read humidity advice on the display unit 12.

The control unit 40 calculates an exercise intensity on the basis of acceleration data detected by the acceleration sensor 19, and calculates an activity amount on the basis of the calculated exercise intensity. Then, the control unit 40 stores an hourly activity amount, a daily activity amount, a weekly activity amount, and a monthly activity amount in the RAM 22.

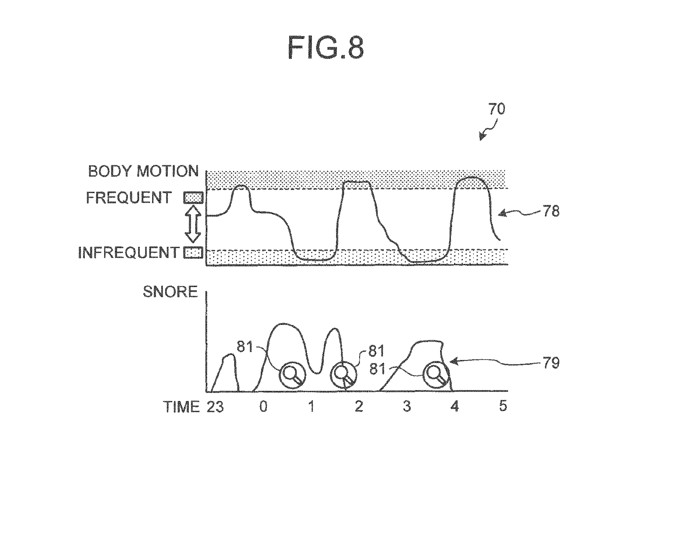

Furthermore, when a tap operation on a graph button on the screen has been detected, the control unit 40 displays a sleep log screen 70 illustrated in FIG. 8, which displays thereon, for example, graphs of the number of body motions, the number of snores, and the number of apneas, etc., on the display unit 12.

When a tap operation on a calendar button on the screen has been detected, the control unit 40 displays a calendar screen on the display unit 12. Furthermore, when a tap operation on an environment check button has been detected, the control unit 40 displays a sleep environment screen, which displays thereon the current illuminance, temperature, and humidity, etc. around the portable terminal 1, on the display unit 12. Moreover, when a tap operation on a bedtime button on the screen has been detected, the control unit 40 activates the sleep log function and displays a wake-up screen on the display unit 12. When a tap operation on a wake-up button on the wake-up screen has been detected, the control unit 40 recognizes that a subject woke up.

On the basis of respective results of the determination by the illuminance determining unit 37, the temperature determining unit 38, and the humidity determining unit 39, the control unit 40 displays the sleep environment screen on the display unit 12. The sleep environment screen includes illuminance advice, temperature advice, humidity advice, and a remeasurement button. The illuminance advice is sleep advice based on a result of determination of current illuminance by the illuminance determining unit 37. The temperature advice is sleep advice based on a result of determination of current temperature by the temperature determining unit 38. The humidity advice is sleep advice based on a result of determination of current humidity by the humidity determining unit 39. The remeasurement button is a button to start remeasurement of the current illuminance, humidity, and temperature.

On the basis of respective results of the determination by the breath determining unit 31, the snore determining unit 32, the apnea determining unit 33, and the body motion determining unit 34, the control unit 40 displays the sleep log screen 70 on the display unit 12. The sleep log screen 70 in FIG. 8 can further include a sleeping time on a specified date, a sleep efficiency, a rank, bedtime, wake-up time, time-variable temperature and humidity, and time-variable sleep and awakening. Furthermore, the sleep log screen 70 includes a time-variable body motion frequency graph 78, a time-variable snore frequency graph 79, and apnea marks 81.

The sleeping time is sleeping hours from sleep-onset time to wake-up time. Incidentally, the wake-up time is the time at which a tap operation on the wake-up button on the wake-up screen has been detected or the time at which subject's action has been detected from a result of detection by the acceleration sensor 19. The sleep efficiency is a ratio of an actual sleeping time to a time from bedtime to wake-up time; the actual sleeping time is derived by subtracting an awakening time from the time from bedtime to wake-up time. The rank denotes evaluation of the sleep efficiency. The bedtime is, for example, the time at which a tap operation on the bedtime button on the bedtime screen has been detected. The wake-up time is, for example, the time at which a tap operation on the wake-up button on the wake-up screen has been detected or the time at which subject's action has been detected from a result of detection by the acceleration sensor 19. The temperature and humidity represent the hourly temperature and humidity in a period from bedtime to wake-up time. The sleep and awakening represent the state transition between sleep and awakening in the period from bedtime to wake-up time. Incidentally, sleep and awakening are displayed in different colors so as to distinguish between the two.

The body motion frequency graph 78 is a graph of the number of hourly body motions in the period from bedtime to wake-up time. The snore frequency graph 79 is a graph of the number of hourly snores in the period from bedtime to wake-up time.

The apnea mark 81 denotes a time period in which an apnea state occurred on the snore frequency graph 79. When a tap operation on an apnea mark 81 on the sleep log screen 70 has been detected, the control unit 40 displays an apnea details screen of an apneas state in a corresponding time period on the display unit 12. Sleep advice represents sleep advice suitable to a condition to be described later. A site button is a button to link to a specified site.

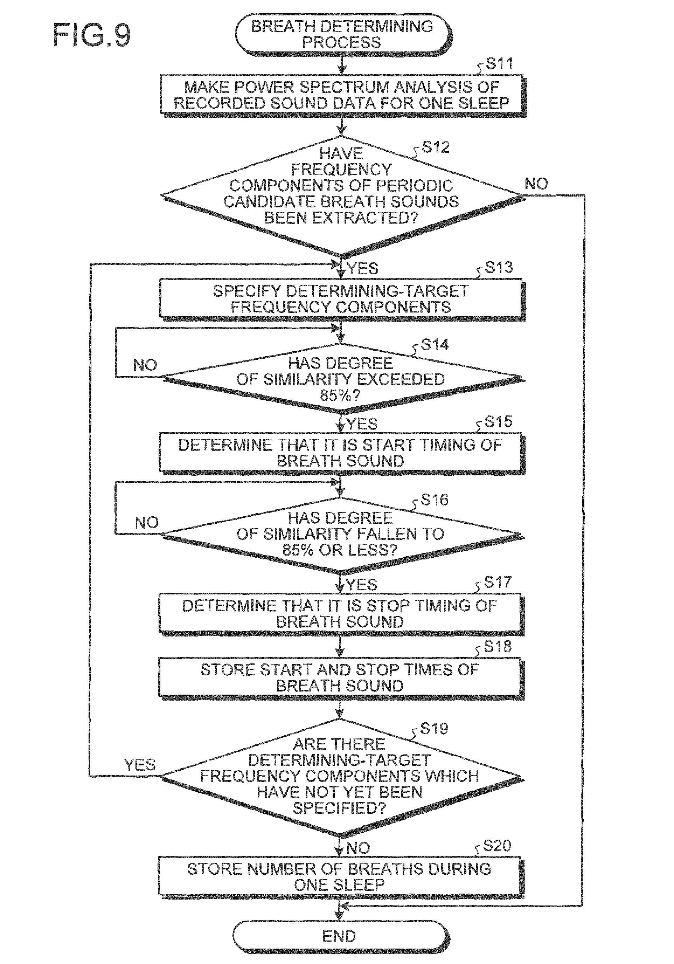

Subsequently, the operation of the portable terminal 1 according to the present embodiment is explained. FIG. 9 is a flowchart illustrating an example of the processing operation of the processor 23 of the portable terminal 1 involved in a breath determining process. The breath determining process is a process of determining breath sounds, which are sounds of subject's breathing during sleep, in recorded sound data for one sleep. In FIG. 9, the breath determining unit 31 of the processor 23 reads out recorded sound data for one sleep from the recorded sound area 22A, and makes a power spectrum analysis of the read recorded sound data (Step S11). The breath determining unit 31 determines whether frequency components of candidate breath sounds of subject's breathing as periodic sounds having similar frequency components have been extracted from the recorded sound data on the basis of a result of the power spectrum analysis (Step S12). Then, when frequency components of candidate breath sounds have been extracted (YES at Step S12), the breath determining unit 31 specifies determining-target frequency components on the time axis in the recorded sound data (Step S13). Incidentally, the breath determining unit 31 sequentially specifies determining-target frequency components on the time axis which are similar to the frequency components of the candidate breath sounds. The breath determining unit 31 determines whether a degree of similarity between the specified determining-target frequency components and the frequency components of the candidate breath sounds has exceeded 85% (Step S14).

When a degree of similarity between the specified determining-target frequency components and the frequency components of the candidate breath sounds has exceeded 85% (YES at Step S14), the breath determining unit 31 determines that it is start timing of a breath sound (Step S15). Furthermore, after having determined the start timing of the breath sound, the breath determining unit 31 determines whether a degree of the similarity between the determining-target frequency components and the frequency components of the candidate breath sounds has fallen to 85% or less (Step S16). When a degree of the similarity between the determining-target frequency components and the frequency components of the candidate breath sounds has fallen to 85% or less (YES at Step S16), the breath determining unit 31 determines that it is stop timing of the breath sound (Step S17). After having determined the stop timing of the breath sound, the breath determining unit 31 stores the start and stop times of the breath sound in the sleep log area 22B (Step S18).

Then, the breath determining unit 31 determines whether there are determining-target frequency components which have not yet been specified (Step S19). When there are no determining-target frequency components which have not yet been specified (NO at Step S19), the breath determining unit 31 stores the number of breaths during the one sleep in the sleep log area 22B (Step S20), and ends the processing operation of the breath determining process illustrated in FIG. 9.

On the other hand, when there are determining-target frequency components which have not yet been specified (YES at Step S19), the breath determining unit 31 returns to Step S13 to specify determining-target frequency components which has not yet been specified. Furthermore, when frequency components of candidate breath sounds have not been extracted (NO at Step S12), the breath determining unit 31 ends the processing operation illustrated in FIG. 9. Moreover, when a degree of similarity between the determining-target frequency components and the frequency components of the candidate breath sounds has not exceeded 85% (NO at Step S14), the breath determining unit 31 repeats Step S14 to determine whether a degree of the similarity has exceeded 85%. Furthermore, when a degree of the similarity between the determining-target frequency components and the frequency components of the candidate breath sounds has not fallen to 85% or less (NO at Step S16), the breath determining unit 31 repeats Step S16 to determine whether a degree of the similarity has fallen to 85% or less.

In the breath determining process illustrated in FIG. 9, the breath determining unit 31 extracts frequency components of periodic candidate breath sounds from recorded sound data of sounds recorded during sleep, and, at timing at which a degree of similarity between determining-target frequency components and the frequency components of the candidate breath sounds has exceeded 85%, determines that it is start timing of a breath sound. Furthermore, after having determined the start timing of the breath sound, at timing at which a degree of the similarity between the determining-target frequency components and the frequency components of the candidate breath sounds has fallen to 85% or less, the breath determining unit 31 determines that it is stop timing of the breath sound. The breath determining unit 31 stores the start and stop times of the breath sound during sleep in the sleep log area 22B. Consequently, the control unit 40 can recognize the respective start and stop times of breath sounds during sleep and the number of hourly breaths during sleep.

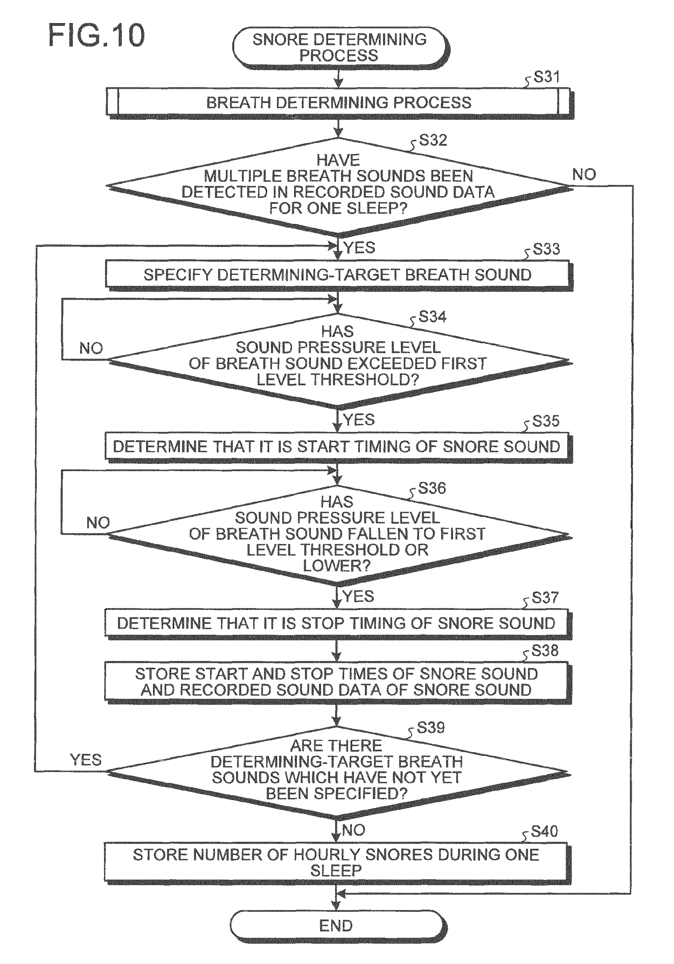

FIG. 10 is a flowchart illustrating an example of the processing operation of the processor 23 of the portable terminal 1 involved in a snore determining process. The snore determining process illustrated in FIG. 10 is a process of determining a snore sound in recorded sound data for one sleep. In FIG. 10, after the execution of the breath determining process illustrated in FIG. 9 (Step S31), the snore determining unit 32 determines whether multiple breath sounds have been detected in recorded sound data for one sleep (Step S32). When multiple breath sounds have been detected (YES at Step S32), the snore determining unit 32 specifies a determining-target breath sound from the multiple breath sounds (Step S33), and determines whether a sound pressure level of the determining-target breath sound has exceeded the first level threshold (Step S34).

When a sound pressure level of the determining-target breath sound has exceeded the first level threshold (YES at Step S34), the snore determining unit 32 determines that it is start timing of a snore sound (Step S35). The snore determining unit 32 determines whether a sound pressure level of the determining-target breath sound has fallen to the first level threshold or lower (Step S36). When a sound pressure level of the determining-target breath sound has fallen to the first level threshold or lower (YES at Step S36), the snore determining unit 32 determines that it is stop timing of the snore sound (Step S37).

After having determined the stop timing of the snore sound, the snore determining unit 32 stores the start and stop times of the snore sound and recorded sound data of the snore sound in the sleep log area 22B (Step S38). The snore determining unit 32 determines whether there are determining-target breath sounds which have not yet been specified (Step S39). When there are no determining-target breath sounds which have not yet been specified (NO at Step S39), the snore determining unit 32 stores the number of hourly snores during the one sleep in the sleep log area 22B (Step S40), and ends the processing operation illustrated in FIG. 10.

When there are determining-target breath sounds which have not yet been specified (YES at Step S39), the snore determining unit 32 returns to Step S33 to specify a determining-target breath sound. When multiple breath sounds have not been detected (NO at Step S32), the snore determining unit 32 ends the processing operation illustrated in FIG. 10.

Furthermore, when a sound pressure level of the determining-target breath sound has not exceeded the first level threshold (NO at Step S34), the snore determining unit 32 repeats Step S34 to determine whether a sound pressure level of the determining-target breath sound has exceeded the first level threshold. Moreover, when a sound pressure level of the determining-target breath sound has not fallen to the first level threshold or lower (NO at Step S36), the snore determining unit 32 repeats Step S36 to determine whether a sound pressure level of the determining-target breath sound has fallen to the first level threshold or lower.

In the snore determining process illustrated in FIG. 10, with respect to each of breath sounds during sleep determined by the breath determining unit 31, the snore determining unit 32 checks a sound pressure level of a breath sound; when a sound pressure level of the breath sound has exceeded the first level threshold, the snore determining unit 32 determines that it is start timing of a snore sound. Furthermore, after having determined the start timing of the snore sound, when a sound pressure level of the breath sound has fallen to the first level threshold or lower, the snore determining unit 32 determines that it is stop timing of the snore sound. The snore determining unit 32 stores the start and stop times of the snore sound during sleep in the sleep log area 22B. Consequently, the snore determining unit 32 determines a snore sound at timing of a breath sound determined by the breath determining unit 31, and therefore, the accuracy of determination of a snore sound is high. Then, the control unit 40 can recognize the respective start and stop times of snore sounds during sleep and the number of hourly snores during sleep.

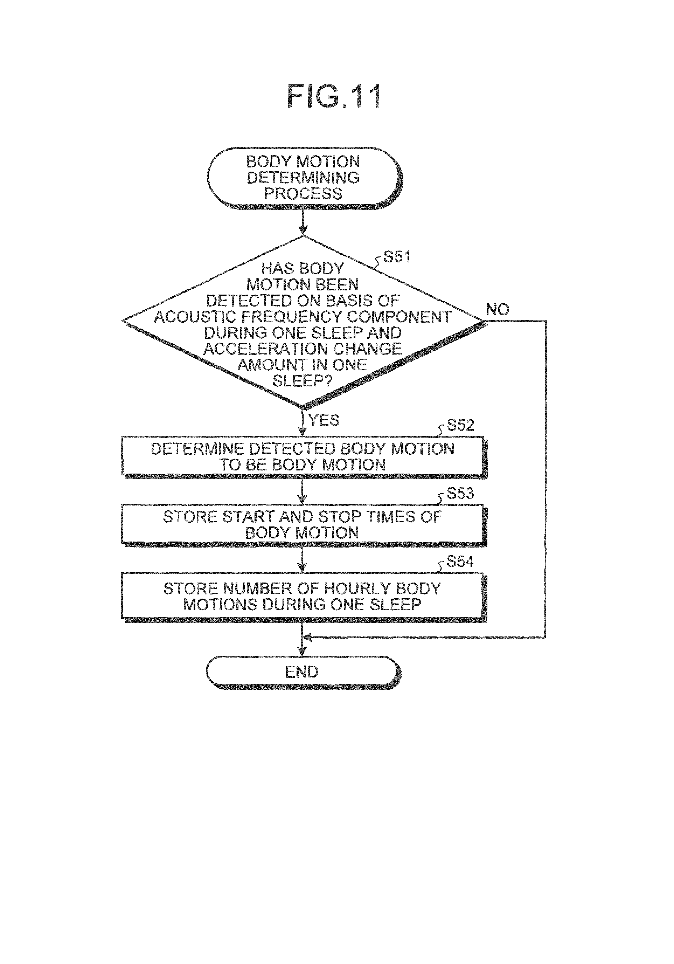

FIG. 11 is a flowchart illustrating an example of the processing operation of the processor 23 of the portable terminal 1 involved in a body motion determining process. The body motion determining process illustrated in FIG. 11 is a process of determining a body motion during one sleep. In FIG. 11, the body motion determining unit 34 of the processor 23 determines whether a body motion has been detected on the basis of an acoustic frequency component during one sleep and an acceleration change amount in the one sleep (Step S51). When acoustic frequency components of a sound produced by a body motion, such as rustling of bedding, has been detected for a duration of a predetermined time in recorded sound data for the one sleep, the body motion determining unit 34 determines that the sound is due to a body motion. Furthermore, when an acceleration change amount during the one sleep has exceeded the body motion threshold G1, the body motion determining unit 34 determines that to be due to a body motion.

When a body motion has been detected (YES at Step S51), the body motion determining unit 34 determines the detected body motion to be a subject's body motion during sleep (Step S52). When having determined the body motion, the body motion determining unit 34 stores the start and stop times of the body motion in the sleep log area 22B (Step S53). Then, the body motion determining unit 34 stores the number of hourly body motions during the one sleep in the sleep log area 22B (Step S54), and ends the processing operation illustrated in FIG. 11.

Furthermore, when any body motion has not been detected (NO at Step S51), the body motion determining unit 34 ends the processing operation illustrated in FIG. 11.

In the body motion determining process illustrated in FIG. 11, the body motion determining unit 34 determines a body motion by detection of frequency components of a sound produced by a body motion, such as rustling of bedding, in recorded sound data for one sleep. Furthermore, when an acceleration change amount during sleep of a vibration transmitted to the portable terminal 1 has exceeded the body motion threshold G1, the body motion determining unit 34 determines that to be due to a body motion. Then, the body motion determining unit 34 stores the start and stop times of the body motion during sleep and the number of hourly body motions during sleep in the sleep log area 22B. Consequently, the control unit 40 can recognize the respective start and stop times of body motions during sleep and the number of hourly body motions during sleep.

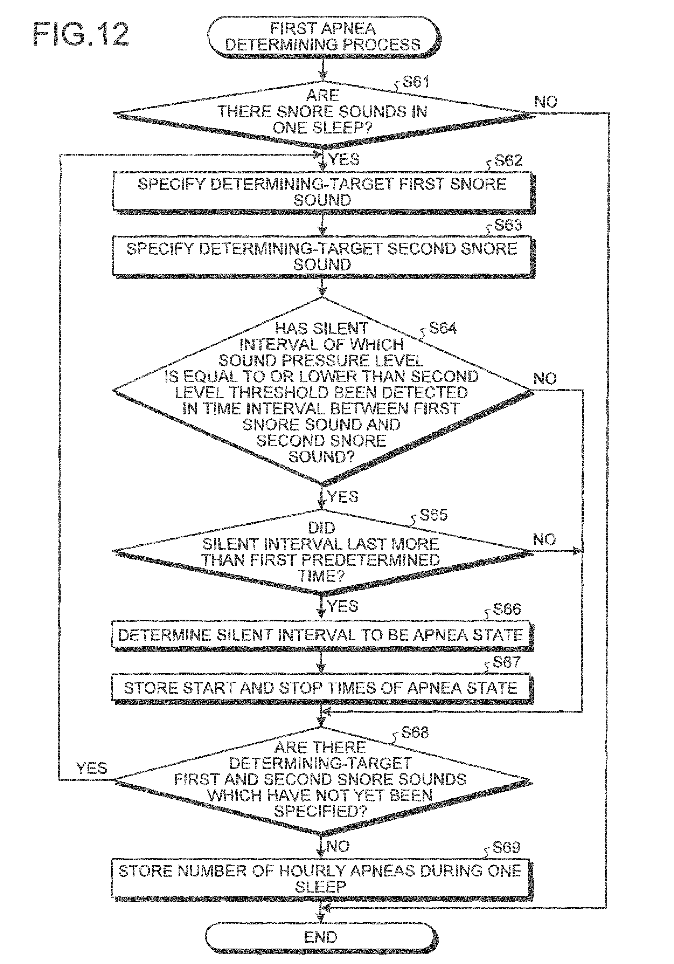

FIG. 12 is a flowchart illustrating an example of the processing operation of the processor 23 of the portable terminal 1 involved in a first apnea determining process. The first apnea determining process illustrated in FIG. 12 is a process of determining an apnea state between consecutive snore sounds. In FIG. 12, the apnea determining unit 33 of the processor 23 determines whether there are multiple snore sounds in one sleep (Step S61). When there are multiple snore sounds in one sleep (YES at Step S61), the apnea determining unit 33 specifies a determining-target first snore sound (Step S62), and specifies a determining-target second snore sound immediately following the first snore sound (Step S63). The apnea determining unit 33 determines whether a silent interval of which the sound pressure level is equal to or lower than a second level threshold has been detected in a time interval between the first snore sound and the second snore sound (Step S64). Incidentally, the second level threshold is, for example, 3 dB.

When a silent interval of which the sound pressure level is equal to or lower than the second level threshold has been detected (YES at Step S64), the apnea determining unit 33 determines whether the silent interval lasted more than a first predetermined time (Step S65). Incidentally, the first predetermined time is, for example, 10 seconds, but the setting of the first predetermined time can be arbitrarily adjusted. When the silent interval lasted more than the first predetermined time (YES at Step S65), the apnea determining unit 33 determines the silent interval to be an apnea state (Step S66). The apnea determining unit 33 stores the start and stop times of the apnea state in the sleep log area 22B (Step S67).

The apnea determining unit 33 determines whether there are determining-target first and second snore sounds which have not yet been specified (Step S68). When there are no determining-target first and second snore sounds which have not yet been specified (NO at Step S68), the apnea determining unit 33 stores the number of hourly apneas during the one sleep in the sleep log area 22B (Step S69), and ends the processing operation illustrated in FIG. 12.

When there are determining-target first and second snore sounds which have not yet been specified (YES at Step S68), the apnea determining unit 33 returns to Step S62 to specify a determining-target first snore sound. When there are not multiple snore sounds in one sleep (NO at Step S61), the apnea determining unit 33 ends the processing operation illustrated in FIG. 12. When a silent interval of which the sound pressure level is equal to or lower than the second level threshold has not been detected in the time interval between the first snore sound and the second snore sound (NO at Step S64), the apnea determining unit 33 moves to Step S68 to determine whether there are first and second snore sounds which have not yet been specified.

Furthermore, when the silent interval did not last more than the first predetermined time (NO at Step S65), the apnea determining unit 33 moves to Step S68 to determine whether there are determining-target first and second snore sounds which have not yet been specified.

In the first apnea determining process illustrated in FIG. 12, when a silent interval has been detected in a time interval between first and second snore sounds out of consecutive snore sounds, the apnea determining unit 33 determines the silent interval to be an apnea state. The apnea determining unit 33 stores the start and stop times of each apnea state with a pattern of a snore sound → an apnea → a snore sound and the number of hourly apneas during sleep in the sleep log area 22B. Consequently, the control unit 40 can recognize the respective start and stop times of apnea states with a pattern of a snore sound → an apnea → a snore sound and the number of hourly apneas during sleep. According to the first apnea determining process described above, an apnea state is determined on the basis of snore sounds produced by a subject, and therefore, the control unit 40 can detect an obstructive apnea.

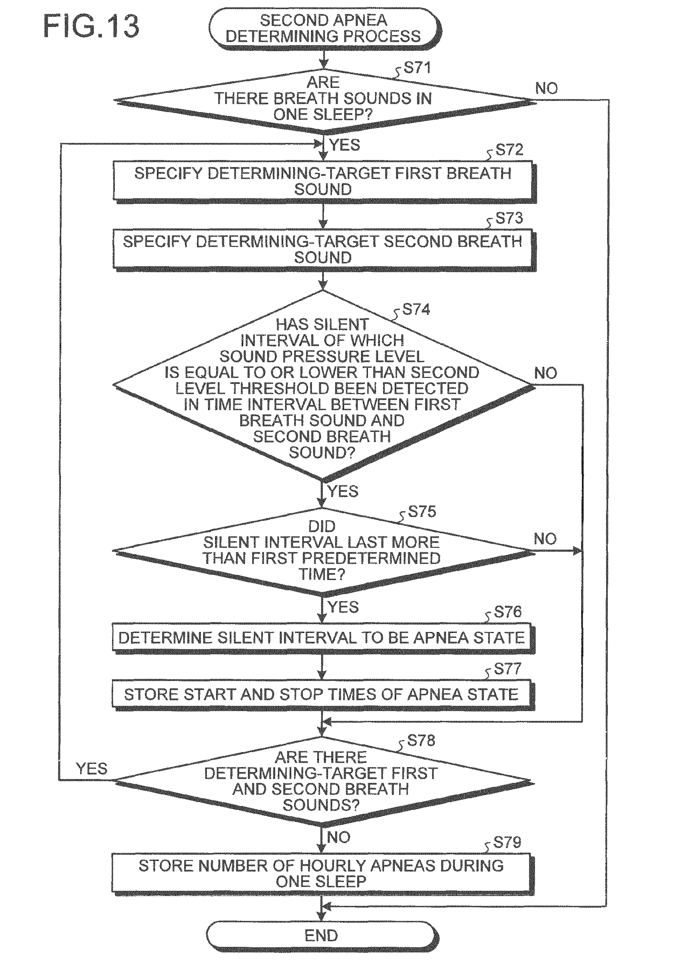

FIG. 13 is a flowchart illustrating an example of the processing operation of the processor 23 of the portable terminal 1 involved in a second apnea determining process. The second apnea determining process illustrated in FIG. 13 is a process of determining an apnea state between consecutive breath sounds. In FIG. 13, the apnea determining unit 33 of the processor 23 determines whether there are multiple breath sounds in one sleep (Step S71). When there are multiple breath sounds in one sleep (YES at Step S71), the apnea determining unit 33 specifies a determining-target first breath sound (Step S72), and specifies a determining-target second breath sound immediately following the first breath sound (Step S73). The apnea determining unit 33 determines whether a silent interval of which the sound pressure level is equal to or lower than a second level threshold has been detected in a time interval between the first breath sound and the second breath sound (Step S74).

When a silent interval of which the sound pressure level is equal to or lower than the second level threshold has been detected (YES at Step S74), the apnea determining unit 33 determines whether the silent interval lasted more than a first predetermined time (Step S75). When the silent interval lasted more than the first predetermined time (YES at Step S75), the apnea determining unit 33 determines the silent interval to be an apnea state (Step S76). The apnea determining unit 33 stores the start and stop times of the apnea state in the sleep log area 22B (Step S77).

The apnea determining unit 33 determines whether there are determining-target first and second breath sounds which have not yet been specified (Step S78). When there are no determining-target first and second breath sounds which have not yet been specified (NO at Step S78), the apnea determining unit 33 stores the number of hourly apneas during the one sleep in the sleep log area 22B (Step S79), and ends the processing operation illustrated in FIG. 13.

When there are determining-target first and second breath sounds which have not yet been specified (YES at Step S78), the apnea determining unit 33 returns to Step S72 to specify a determining-target first breath sound. When there are not multiple breath sounds in one sleep (NO at Step S71), the apnea determining unit 33 ends the processing operation illustrated in FIG. 13. When a silent interval of which the sound pressure level is equal to or lower than the second level threshold has not been detected in the time interval between the first breath sound and the second breath sound (NO at Step S74), the apnea determining unit 33 moves to Step S78 to determine whether there are determining-target first and second breath sounds which have not yet been specified.

Furthermore, when the silent interval did not last more than the first predetermined time (NO at Step S75), the apnea determining unit 33 moves to Step S78 to determine whether there are determining-target first and second breath sounds which have not yet been specified.

In the second apnea determining process illustrated in FIG. 13, when a silent interval has been detected in a time interval between first and second breath sounds out of consecutive breath sounds, the apnea determining unit 33 determines the silent interval to be an apnea state. The apnea determining unit 33 stores the start and stop times of each apnea state with a pattern of a breath sound → an apnea → a breath sound and the number of hourly apneas during sleep in the sleep log area 22B. Consequently, the control unit 40 can recognize the respective start and stop times of apnea states with a pattern of a breath sound → an apnea → a breath sound and the number of hourly apneas during sleep. According to the second apnea determining process described above, an apnea state is determined on the basis of breath sounds produced by a subject during sleep, and therefore, the control unit 40 can detect a central apnea.

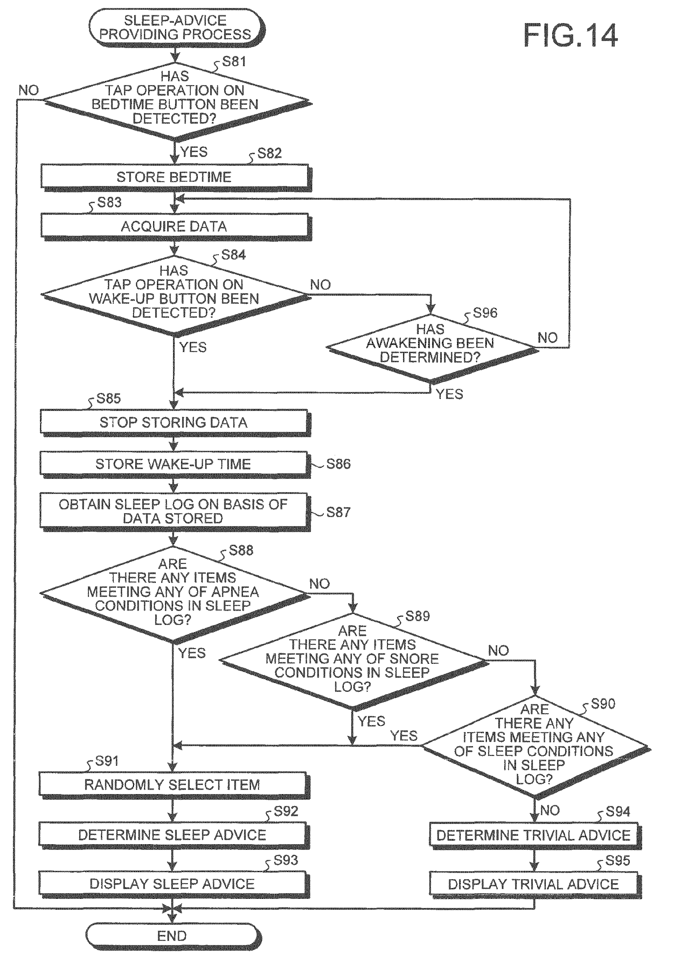

FIG. 14 is a flowchart illustrating an example of the processing operation of the processor 23 of the portable terminal 1 involved in a sleep-advice providing process. The sleep-advice providing process illustrated in FIG. 14 is a process of displaying advice according to contents of a sleep log on the display unit 12. In FIG. 14, the control unit 40 of the processor 23 determines whether a tap operation on the bedtime button on the bedtime screen has been detected (Step S81). When a tap operation on the bedtime button has been detected (YES at Step S81), the control unit 40 stores the bedtime in the sleep log area 22B (Step S82). The control unit 40 acquires various data relevant to a sleep log (Step S83). Incidentally, the various data relevant to a sleep log are, for example, recorded sound data of sounds during sleep picked up through the microphone 13 and data of an acceleration change amount collected through the acceleration sensor 19, etc.

The control unit 40 determines whether a tap operation on the wake-up button on the wake-up screen has been detected (Step S84). When a tap operation on the wake-up button has been detected (YES at Step S84), the control unit 40 stops the operation to acquire data relevant to a sleep log (Step S85), and stores the wake-up time in the sleep log area 22B (Step S86). The control unit 40 obtains a sleep log on the basis of the various data stored in the RAM 22 (Step S87). Incidentally, the sleep log is, for example, data obtained in the breath determining process, the snore determining process, the body motion determining process, the first apnea determining process, and the second apnea determining process. That is, for example, various data on the respective start and stop times of breath sounds, the respective start and stop times of snore sounds, the number of snores, the respective start and stop times of body motions, the number of body motions, the respective start and stop times of apnea states, and the number of apneas, etc.

The control unit 40 determines whether there are any items meeting any of apnea conditions in the sleep log (Step S88). Incidentally, an item meeting an apnea condition is, for example, a condition item using the number of apneas. The control unit 40 selects sleep advice corresponding to the apnea condition from the table 21A. For example, in a case of an apnea condition that the number of hourly apneas during one sleep is five or more, the control unit 40 selects sleep advice of "You often doze off, don't you? If you do, please click a link button below. ((Click here))" from the table 21A. Furthermore, for example, in a case of apnea conditions that a body motion is made within 30 seconds after the occurrence of an apnea state and the total number of body motions during sleep is ten or more, the control unit 40 selects sleep advice of "Frequent body motions may be because you're not sleeping well. If so, please click a link button below. ((Click here))" from the table 21A. Moreover, in a case of apnea conditions that subject's weight is 80 Kg or more, the number of snores is 20 or more, and the number of apneas is 20 or more, the control unit 40 selects sleep advice of "Do you know sleep apnea syndrome? A plump person is prone to have sleep apnea syndrome." from the table 21A. Incidentally, as for the condition of weight or the like, if a user registers his/her weight, height, and vital data, such as blood pressure and pulse, on a specified site, and the condition of weight or the like can be acquired from this specified site.

When there are no items meeting any of apnea conditions in the sleep log (NO at Step S88), the control unit 40 determines whether there are any items meeting any of snore conditions in the sleep log (Step S89). Incidentally, an item meeting a snore condition is, for example, a condition item using the number of snores or the like. The control unit 40 selects sleep advice corresponding to the snore condition from the table 21A. For example, in a case of a snore condition that the number of snores during one sleep is one or more, the control unit 40 selects sleep advice of "You seemed to snore yesterday. Did you know it?" from the table 21A. Furthermore, for example, in a case of snore conditions that the number of snores during one sleep is ten or more and a total duration of snore sounds during the one sleep is 60 minutes or more, the control unit 40 selects sleep advice of "You snored." or the like from the table 21A.

Furthermore, when there are no items meeting any of snore conditions in the sleep log (NO at Step S89), the control unit 40 determines whether there are any items meeting any of sleep conditions in the sleep log (Step S90). Incidentally, an item meeting a sleep condition is, for example, a condition item using a condition related to sleep, such as the number of awakenings. The control unit 40 selects sleep advice corresponding to the sleep condition from the table 21A. For example, in a case of sleep conditions that a sleeping time is less than 5 hours and the number of awakenings during sleep is five or more, the control unit 40 selects sleep advice of "You wake a lot during the night, don't you? If you do, please click a link button below. 〈〈Click here〉〉" from the table 21A. Furthermore, for example, in a case of sleep conditions that it takes 60 minutes or more from bedtime to sleep-onset time and a total daytime activity amount is 1.0 EX or less, the control unit 40 selects sleep advice of "It takes a long time from when you go to bed till when you fall asleep, doesn't it? Why don't you exercise more to increase a daytime activity amount." from the table 21A. Moreover, for example, in a case of a sleep condition that a sleeping time has been 4 hours or less for three consecutive days, the control unit 40 selects sleep advice of "You haven't been getting enough sleep lately. Lack of sleep is a great enemy to dieting!" from the table 21A.

When there are items meeting any of apnea conditions (YES at Step S88), the control unit 40 randomly selects an item meeting the condition (Step S91). Incidentally, when there are multiple items meeting any of conditions, the control unit 40 randomly selects one item from the multiple items. Furthermore, when there are items meeting any of snore conditions in the sleep log (YES at Step S89) or when there are items meeting any of sleep conditions in the sleep log (YES at Step S90), the control unit 40 moves to Step S91 to randomly select an item meeting the condition. The control unit 40 determines sleep advice corresponding to the randomly-selected item from the table 21A (Step S92), and displays the determined sleep advice on the display unit 12 (Step S93), and ends the processing operation illustrated in FIG. 14.

Furthermore, when there are no items meeting any of sleep conditions in the sleep log (NO at Step S90), the control unit 40 determines trivial advice from the table 21A (Step S94). Incidentally, the trivial advice is for introducing bits of knowledge for achieving comfortable sleep, and contains, for example, "There are two stages of sleep: REM sleep and non-REM sleep. REM sleep is deep sleep, and non-REM sleep is light sleep.", etc. Then, the control unit 40 displays the determined trivial advice on the display unit 12 (Step S95), and ends the processing operation illustrated in FIG. 14.

Furthermore, when a tap operation on the bedtime button has not been detected (NO at Step S81), the control unit 40 ends the processing operation illustrated in FIG. 14. Moreover, when a tap operation on the wake-up button has not been detected (NO at Step S84), the awakening determining unit 36 determines whether awakening has been detected by subject's wake-up action (Step S96).

When having determined awakening from the wake-up action (YES at Step S96), the awakening determining unit 36 moves to Step S85 to stop the operation to acquire data relevant to the sleep log. Furthermore, when not having determined awakening from the wake-up action (NO at Step S96), the awakening determining unit 36 moves to Step S83 to continue the operation to acquire data relevant to the sleep log.

In the sleep-advice providing process illustrated in FIG. 14, when a sleep log in a period from detection of a tap operation on the bedtime button to wake-up meets any of apnea conditions, sleep advice corresponding to the apnea condition is selected and displayed on the display unit 12. Consequently, a user can recognize the occurrence of an apnea state during sleep by viewing the sleep advice.

In the sleep-advice providing process, when a sleep log in a period from detection of a tap operation on the bedtime button to wake-up meets any of snore conditions, sleep advice corresponding to the snore condition is selected and displayed on the display unit 12. Consequently, a user can recognize the occurrence of snoring during sleep by viewing the sleep advice on snoring.

In the sleep-advice providing process, when a sleep log in a period from detection of a tap operation on the bedtime button to wake-up meets any of sleep conditions, sleep advice corresponding to the sleep condition is selected and displayed on the display unit 12. Consequently, a user can recognize disorder during sleep by viewing the sleep advice.

In the sleep-advice providing process, when a sleep log in a period from detection of a tap operation on the bedtime button to wake-up does not meet any of apnea conditions, snore conditions, and sleep conditions, trivial advice on sleep is selected and displayed on the display unit 12. Consequently, a user can gain bits of knowledge of sleep by viewing the trivial advice.

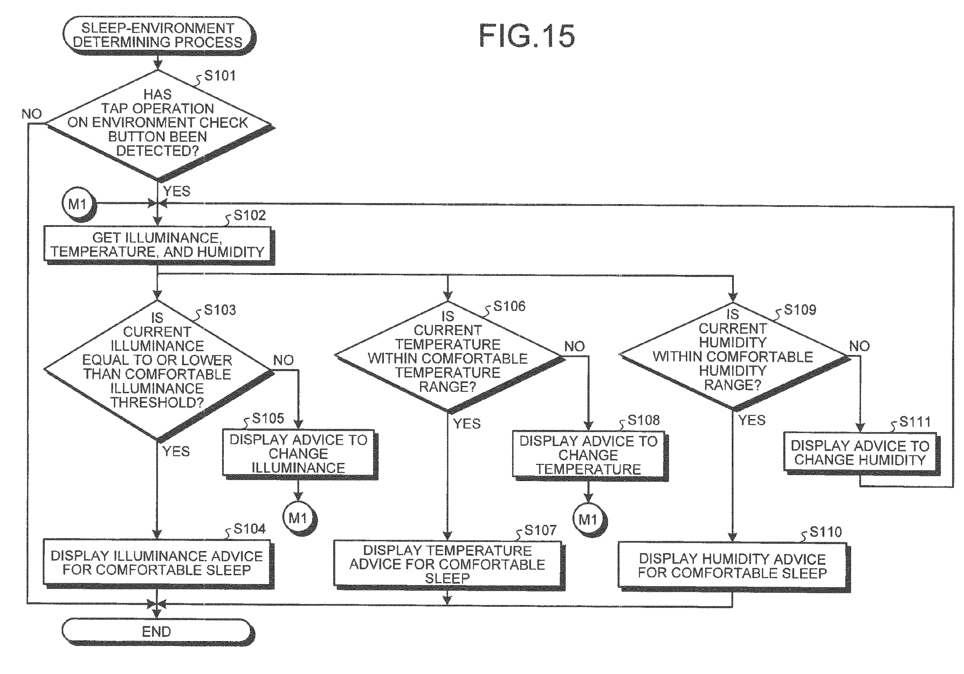

FIG. 15 is a flowchart illustrating an example of the processing operation of the processor 23 of the portable terminal 1 involved in a sleep-environment determining process. The sleep-environment determining process illustrated in FIG. 15 is a process of displaying environment advice according to current sleep environment. In FIG. 15, the control unit 40 of the processor 23 determines whether a tap operation on the environment check button has been detected (Step S101). When a tap operation on the environment check button has been detected (YES at Step S101), the control unit 40 gets illuminance, temperature, and humidity through the illuminance sensor 18, the temperature sensor 16, and the humidity sensor 17 (Step S102).

After illuminance, temperature, and humidity are obtained at Step S102, the illuminance determining unit 37 determines whether the current illuminance is equal to or lower than the comfortable illuminance threshold suitable for sleep (Step S103). When the current illuminance is equal to or lower than the comfortable illuminance threshold (YES at Step S103), the illuminance determining unit 37 displays illuminance advice for comfortable sleep on the display unit 12 (Step S104), and ends the processing operation illustrated in FIG. 15. On the other hand, when the current illuminance is not equal to or lower than the comfortable illuminance threshold (NO at Step S103), the illuminance determining unit 37 displays illuminance advice on prompting a change of the illuminance on the display unit 12 (Step S105), and returns to Step S102 as indicated by M1 in FIG. 15 to get illuminance, temperature, and humidity.

Furthermore, after illuminance, temperature, and humidity are obtained at Step S102, the temperature determining unit 38 determines whether the current temperature is within the comfortable temperature range suitable for sleep (Step S106). When the current temperature is within the comfortable temperature range (YES at Step S106), the temperature determining unit 38 displays temperature advice for comfortable sleep on the display unit 12 (Step S107), and ends the processing operation illustrated in FIG. 15. On the other hand, when the current temperature is not within the comfortable temperature range (NO at Step S106), the temperature determining unit 38 displays temperature advice on prompting a change of the temperature on the display unit 12 (Step S108), and returns to Step S102 as indicated by M1 in FIG. 15 to get illuminance, temperature, and humidity.

After illuminance, temperature, and humidity are obtained at Step S102, the humidity determining unit 39 determines whether the current humidity is within the comfortable humidity range suitable for sleep (Step S109). When the current humidity is within the comfortable humidity range (YES at Step S109), the humidity determining unit 39 displays humidity advice for comfortable sleep on the display unit 12 (Step S110), and ends the processing operation illustrated in FIG. 15. On the other hand, when the current humidity is not within the comfortable humidity range (NO at Step S109), the humidity determining unit 39 displays humidity advice on prompting a change of the humidity on the display unit 12 (Step S111), and returns to Step S102 to get illuminance, temperature, and humidity.

Furthermore, when a tap operation on the environment check button on the display screen has not been detected (NO at Step S101), the control unit 40 ends the processing operation illustrated in FIG. 15.

In the sleep-environment determining process illustrated in FIG. 15, when current illuminance detected by the illuminance sensor 18 of the portable terminal 1 is equal to or lower than the comfortable illuminance threshold, the illuminance determining unit 37 displays illuminance advice for comfortable sleep on the display unit 12. Consequently, a user can recognize that the current illuminance is comfortable to sleep by viewing the illuminance advice.

When the current illuminance is not equal to or lower than the comfortable illuminance threshold, the illuminance determining unit 37 displays illuminance advice on prompting an adjustment of current illuminance on the display unit 12. Consequently, a user can recognize that the current illuminance is not suitable for sleep by viewing the illuminance advice.

When current temperature detected by the temperature sensor 16 of the portable terminal 1 is within the comfortable temperature range, the temperature determining unit 38 displays temperature advice for comfortable sleep on the display unit 12. Consequently, a user can recognize that the current temperature is comfortable to sleep by viewing the temperature advice.

When the current temperature is not within the comfortable temperature range, the temperature determining unit 38 displays temperature advice on prompting an adjustment of current temperature on the display unit 12. Consequently, a user can recognize that the current temperature is not suitable for sleep by viewing the temperature advice.

When current humidity detected by the humidity sensor 17 of the portable terminal 1 is within the comfortable humidity range, the humidity determining unit 39 displays humidity advice for comfortable sleep on the display unit 12. Consequently, a user can recognize that the current humidity is comfortable to sleep by viewing the humidity advice.

When the current humidity is not within the comfortable humidity range, the humidity determining unit 39 displays humidity advice on prompting an adjustment of current humidity on the display unit 12. Consequently, a user can recognize that the current humidity is not suitable for sleep by viewing the humidity advice.

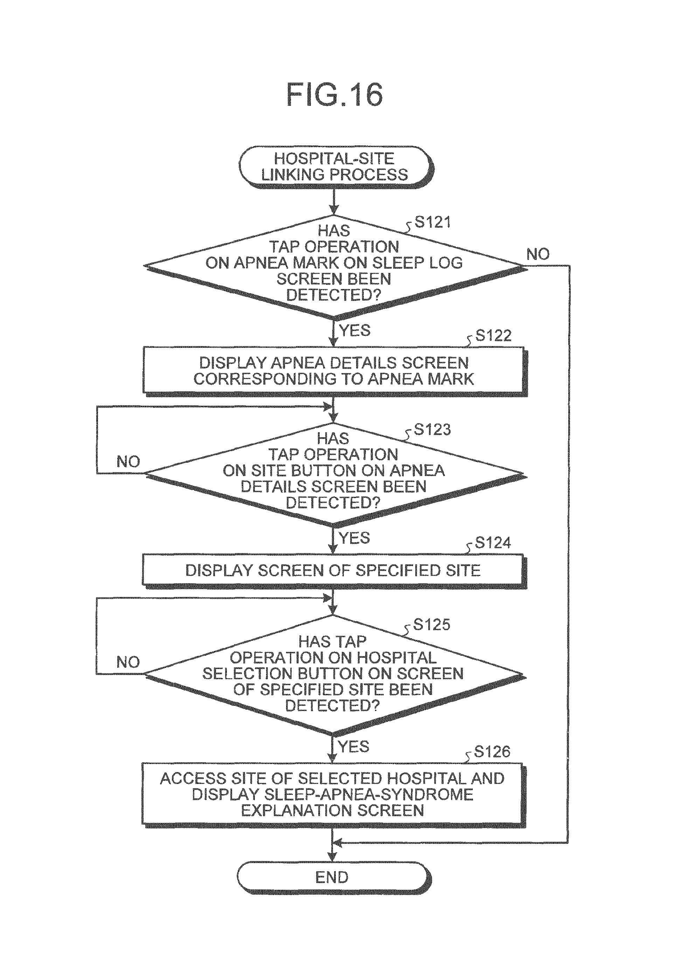

FIG. 16 is a flowchart illustrating an example of the processing operation of the processor 23 of the portable terminal 1 involved in a hospital-site linking process. The hospital-site linking process illustrated in FIG. 16 is a process of displaying the details of an apnea in response to a tap operation on an apnea mark 81 on the sleep log screen 70 and then leading a user to a hospital site.

In FIG. 16, the control unit 40 of the processor 23 determines whether a tap operation on an apnea mark 81 on the sleep log screen 70 illustrated in FIG. 8 has been detected (Step S121). When a tap operation on an apnea mark 81 has been detected (YES at Step S121), the control unit 40 reads out data on an apnea corresponding to the apnea mark 81 from the sleep log area 22B and displays an apnea details screen on the display unit 12 (Step S122). Incidentally, the apnea details screen includes, for example, a schematic diagram illustrating a duration of an apnea between snore sounds, i.e., a period of time from the start time of an apnea between snore sounds to the stop time of the apnea, a comment on the apnea, and a specified site button for accessing a specified site.

The control unit 40 determines whether a tap operation on the specified site button on the apnea details screen has been detected (Step S123). When a tap operation on the specified site button has been detected (YES at Step S123), the control unit 40 accesses a specified site and displays a screen of the specified site on the display unit 12 (Step S124). Furthermore, the control unit 40 determines whether a tap operation on a hospital selection button on the screen of the specified site has been detected (Step S125).

When a tap operation on the hospital selection button on the screen of the specified site has been detected (YES at Step S125), the control unit 40 displays a sleep-apnea-syndrome explanation screen of a site of a selected hospital on the display unit 12 (Step S126). Then, the control unit 40 ends the processing operation illustrated in FIG. 16.

When a tap operation on an apnea mark 81 on the sleep log screen 70 has not been detected (NO at Step S121), the control unit 40 ends the processing operation illustrated in FIG. 16. Furthermore, when a tap operation on the specified site button on the apnea details screen has not been detected (NO at Step S123), the control unit 40 repeats Step S123 to determine whether a tap operation on the specified site button has been detected. When a tap operation on the hospital selection button on the screen of the specified site has not been detected (NO at Step S125), the control unit 40 repeats Step S125 to determine whether a tap operation on the hospital selection button has been detected.

In the hospital-site linking process illustrated in FIG. 16, when a tap operation on an apnea mark 81 on the sleep log screen 70 has been detected, the control unit 40 displays an apnea details screen on the display unit 12. Consequently, a user can recognize detailed information on an apnea state by viewing the apnea details screen.

Furthermore, when a tap operation on a specified site button on the apnea details screen has been detected, the control unit 40 displays a screen of a specified site on the display unit 12. Consequently, a user can recognize a hospital well-versed in sleep apnea syndrome by viewing the screen of the specified site.

Moreover, when a tap operation on a hospital selection button on the screen of the specified site has been detected, the control unit 40 displays a sleep-apnea-syndrome explanation screen of a site of a selected hospital on the display unit 12. Consequently, a user can recognize the details of sleep apnea syndrome by viewing the explanation screen of the hospital.

In the present embodiment, when sounds having similar frequency components has been periodically detected in recorded sound data of sounds during sleep, the breath determining unit 31 determines the detected sounds to be breath sounds. Furthermore, when a silent interval has been detected between consecutive breath sounds determined, the apnea determining unit 33 determines the silent interval to be an apnea state. Consequently, by the use of breath timing, a state likely to be an apnea state between consecutive breath sounds can be determined with a high degree of accuracy.

In the present embodiment, when sounds having similar frequency components has been periodically detected in recorded sound data of sounds during sleep, the breath determining unit 31 determines the detected sounds to be breath sounds. Furthermore, when a sound pressure level of a determined breath sound has exceeded the first level threshold, the snore determining unit 32 determines the breath sound to be a snore sound. Consequently, even when a noise resemblant to a snore has occurred between consecutive breath sounds, the noise is not determined to be a snore; therefore, it is possible to avoid erroneous determination of a snore sound.

Furthermore, in the present embodiment, when a silent interval has been detected between consecutive snore sounds determined, the apnea determining unit 33 determines the silent interval to be an apnea state. Consequently, by the use of snore timing, a state likely to be an apnea state between consecutive snore sounds can be determined with a high degree of accuracy.

In the present embodiment, the control unit 40 displays the sleep log screen 70 based on the sleep log on the display unit 12. Consequently, a user can recognize information about sleep as well as, for example, the number of body motions, the number of snores, and the number of apneas by viewing the sleep log screen 70. Furthermore, the user can recognize sleep advice on the sleep log.

Moreover, by making a tap operation on an apnea mark 81 on the sleep log screen 70, the user can recognize an apnea details screen displaying thereon the details of an apnea occurred in a time period corresponding to the apnea mark 81. Furthermore, by making a tap operation on a snore mark on the sleep log screen 70, the user can listen to a recorded snore sound in a time period corresponding to the snore mark. Moreover, the user can recognize advice on a current sleep log by viewing sleep advice on the sleep log screen 70. Furthermore, by making a tap operation on a specified site button on the sleep log screen 70, the user can easily access a specified site. Moreover, after having accessed the specified site, by making a tap operation on a hospital selection button on the screen of the specified site, the user can view a sleep explanation screen of a site of a selected hospital, thereby recognizing a symptom of sleep. Consequently, according to contents of the sleep log, the user can be aware of, for example, sleep apnea, syndrome.

Incidentally, in the above-described embodiment, there is described the portable terminal 1, such as a smartphone, as an example; however, the portable terminal 1 can be, for example, a cellular phone, a portable game terminal, a tablet terminal, and a portable terminal without a communication function, etc.

Furthermore, in the above-described embodiment, the calendar screen is displayed on the display unit 12 when a tap operation on the calendar button has been detected. At this time, the rank of a daily sleep log is displayed on the calendar screen on a monthly basis.

Moreover, in the above-described embodiment, the day-by-day sleep log screen 70 is displayed on the display unit 12; alternatively, the portable terminal 1 can be configured to tally the number of snores, the number of apneas, and the number of body motions, etc. on a weekly or monthly basis and display a week-by-week or month-by-month sleep log screen on the display unit 12.

Furthermore, in the above-described embodiment, the presence or absence of a body motion is determined by using both an acoustic frequency component on the occurrence of a body motion and an acceleration component on the occurrence of the body motion; alternatively, a body motion can be determined by using either one of the two.

Moreover, components of each unit illustrated in the drawings do not always have to be physically configured as illustrated in the drawings. Namely, the specific forms of division and integration of units are not limited to those illustrated in the drawings, and all or some of the units can be configured to be functionally or physically divided or integrated in arbitrary units depending on respective loads and use conditions, etc.

Furthermore, all or any part of various processing functions implemented in each apparatus can be executed on a central processing unit (CPU) (or a microcomputer, such as a micro processing unit (MPU) and a micro controller unit (MCU)). Moreover, it goes without saying that all or any part of the processing functions can be executed on a program that the CPU (or the microcomputer, such as an MPU and an MCU) analyzes and executes or on wired logic hardware.

Incidentally, the various processes described in the present embodiment can be realized by causing an electronic device to execute a program prepared in advance. An example of an electronic device that executes a program having the same functions as those described in the above embodiment is explained below. FIG. 17 is an explanatory diagram illustrating an example of an electronic device that executes an apnea determining program.

An electronic device 100 illustrated in FIG. 17, which executes the apnea determining program, includes a ROM 110, a RAM 120, an input-output interface 130, a display unit 140, and a processor 150.

The apnea determining program, which fulfills the same functions as those described in the above embodiment, has been stored in the ROM 110 in advance. Incidentally, the apnea determining program can be stored not in the ROM 110 but on a recording medium that a drive not illustrated) can read. Furthermore, as the recording medium, for example, a portable recording medium, such as a CD-ROM, a DVD, a USB flash drive, and an SD card, or a semiconductor memory, such as a flash memory, can be used. As illustrated in FIG. 17, the apnea determining program includes a first determining program 110A and a second determining program 110B. Incidentally, the programs 110A and 110B can be arbitrarily integrated or divided.

The processor 150 reads out these programs 110A and 110B from the ROM 110, and executes the read programs 110A and 110B on the RAM 120. Then, the processor 150 makes the programs 110A and 110B operate as a first determining process 150A and a second determining process 150B, respectively, as illustrated in FIG. 17.

When sounds having similar frequency components have been periodically detected in picked-up sounds, the processor 150 determines the detected sounds to be breath sounds. Furthermore, when a silent interval has been detected between the determined consecutive breath sounds, the processor 150 determines the detected silent interval to be an apnea state. Consequently, a state likely to be an apnea state can be determined with a high degree of accuracy.

## Claims

1. An apnea determining program causing a processor(23) of an electronic device(1) to execute:
first determining(31), when having detected sounds having similar frequency components periodically in picked-up sounds, the detected sounds to be breath sounds; and
second determining(33), when having detected a silent interval between consecutive breath sounds determined, the detected silent interval to be an apnea state.

2. The apnea determining program according to claim 1, wherein
the second determining includes:
determining, when sound pressure of a determined breath sound has exceeded a predetermined threshold, the breath sound to be a snore sound; and
determining, when having detected a silent interval between consecutive snore sounds determined, the detected silent interval to be an apnea state.

3. An apnea determining apparatus(1) comprising:
a first determining unit(31) that determines, when sounds having similar frequency components have been periodically detected in picked-up sounds, the detected sounds to be breath sounds; and
a second determining unit(33) that determines, when a silent interval has been detected between consecutive breath sounds determined, the detected silent interval to be an apnea state.

4. An apnea determining method of an electronic device(1), the apnea determining method comprising:
determining, when having detected sounds having similar frequency components periodically in picked-up sounds, the detected sounds to be breath sounds; and
determining, when having detected a silent interval between consecutive breath sounds determined, the detected silent interval to be an apnea state.
